Europäisches Patentamt

European Patent Office

Office européen des brevets

(19)

(11) Publication number: **0 004 694**
A2

(12) **EUROPEAN PATENT APPLICATION**

(21) Application number: **79200174.5**

(22) Date of filing: **10.04.79**

(51) Int. Cl.²: **A 61 N 5/01**

(30) Priority: **12.04.78 SE 7804096**

(43) Date of publication of application:
**17.10.79 Bulletin 79/21**

(84) Designated Contracting States:
**CH DE FR GB IT SE**

(71) Applicant: **Svenska Philipsföretagen AB Patent**
**S-11584 Stockholm(SE)**

(84) Designated Contracting States:
**SE**

(71) Applicant: **N.V. Philips' Gloeilampenfabrieken**
**Emmasingel 29**
**NL-5611 AZ Eindhoven(NL)**

(84) Designated Contracting States:
**CH DE FR GB IT**

(72) Inventor: **Bermas, Gunnar**
**c/o INT. OCTROOIBUREAU B.V. 6, Prof. Holstlaan**
**NL-5656 AA Eindhoven(NL)**

(74) Representative: **Scheele, Edial François**
**INTERNATIONAAL OCTROOIBUREAU B.V. Prof.**
**Holstlaan 6**
**NL-5656 AA Eindhoven(NL)**

(54) **Medical irradiating apparatus.**

(57) In an X-ray or electron irradiation apparatus which is provided with a radiation source, a patient table, and at least one pair of coupled aperture elements disposed between the radiation source and the patient, an interlock is provided between the movement of the aperture elements and the patient table, so that during irradiation an asymmetrical radiation distribution can be obtained. In a conventional irradiation apparatus this merely requires the addition of a suitable control device for the existing movement mechanisms for adjusting an irradiation field on the patient.

Fig.5

2-III-PHZ 78-004

**EP 0 004 694 A2**

23-2-1979                    1              PHZ 78 004

"Medical irradiating apparatus"

The invention relates to a medical irradiation apparatus, provided with a radiation source, means for directing a radiation beam to be produced by the radiation source, and aperture system with at least two aperture elements which are adjustable relative to each other, and a patient table which is adjustable relative to the radiation source.

Such an irradiation apparatus serves to concentrate a radiation dose within a specific body area so as to destroy tissues in said area, whilst surrounding tissues receive a minimal radiation dose. It is therefore important that an effective irradiation area, which area is for example defined as a volume within which the radiation dose is 90% of a maximum value, is adapted to the shape of that part of the body, for example a tumour, to be destroyed.

In order to obtain a high concentration of radiation within a specific body area, irradiation is effected from a number of different directions, usually two to four, the total radiation dose being a maximum in a point which receives a high radiation energy from each direction of irradiation. Thus, it is possible to obtain a sharp boundary between an area within which tissue is to be destroyed and a surrounding area where the tissue is not

0004694

damaged permanently. As radiation electromagnetic radiation
(X-rays, radiation) as well as corpuscular radiation (electrons, neutrons, etc.) may be used.

For optimum results it is desirable that the
radiation distribution for each direction of irradiation
is adapted to the shape of the area to be destroyed, viewed
from said direction of irradiation.

. The means employed for adjusting the irradiation field comprise the said aperture elements, which in
practice take the form of heavy, generally rectangular,
blocks of lead. Usually two pairs of such lead blocks are
employed which are disposed perpendicular to each other,
said blocks thus defining a rectangular irradiation field.
Generally the blocks are mechanically interconnected in
pairs and when the size of the irradiation field is to be
changed the blocks of each pair are moved symmetrically
relative to a central axis of the radiation source, i.e.
both blocks of a pair are always situated at equal distances from the said axis. Such irradiation apparatus produces a radiation distribution which is symmetrical relative
to the axis of the radiation source and is known in the form
of a linear accelerator from United States Patent Specification no. 3,322,950.

In order to adapt the radiation to the actual
shape of for example a tumour to be destroued or, in specific cases, to protect highly sensitive parts of the body
it is often required to have an asymmetrical radiation
distribution, i.e. a distribution which exhibits a maximum
radiation at one edge of the irradiation field, decreasing
towards the opposite edge of the field. This is generally
realized by means of wedge-shaped absorption bodies which
are arranged in the radiation path between the radiation
source and the patient. For adjusting the desired decrease
of the radiation a set of such wedges with different wedge
angles is then employed.

This is a static solution which, owing to the
large differences between the absorption wedge angles,
merely allows a limited coarse adjustment to be made.

It is the object of the invention to enable an asymmetrical radiation distribution to be obtained in a simple manner and for any arbitrary direction. According to the invention this is achieved in that an irradiation apparatus of the type mentioned in the preamble is characterized in that a movement mechanism for the patient table and at least one of the aperture elements is controllable by a control device which couples the two movements.

An irradiation apparatus in accordance with the invention for obtaining an arbitrary radiation distribution, such as an asymmetrical radiation distribution has the advantage that this can be realized with a minimum of additional motion mechanisms, which render such an apparatus substantially more expensive and more difficult to operate. The only addition is a comparatively simple control and synchronizing function which is added to the existing motion mechanism. Despite its simplicity the invention enables an arbitrary dose distribution to be adjusted, not only in a direction of movement which coincides with the direction of movement of the pairs of aperture elements, but also in a direction transverse thereto, coinciding with the direction of movement of a second pair of aperture elements, because the table is generally also adjustable in two directions perpendicular to each other.

A preferred embodiment of the invention is characterized in that the coupling in the moving mechanism to the aperture element and the patient table results in an equal and equally directed angular speed, viewed from the radiation source. This coupling results in an asymmetrical dose distribution in the patient, in spite of the fact that the apparatus itself is symmetrical.

A further preferred embodiment is characterized in that the radiation source with the aperture system is rotatable about the patient table and the movement of an aperture element in this respect is adjustable depending on the angle.

In order to obtain a synchronous movement of the table and at least one of the aperture elements, an

0004694

irradiation apparatus in accordance with the invention may be equipped with detectors for supplying signals which are a measure of the instantaneous position of the patient table and the aperture element respectively relative to a reference position.

It is very important that the desired radiation distribution is obtained exactly. This can be achieved with the preferred embodiment in which each of the simultaneous movements of the table and the aperture elements is measured with position detectors, for example potentiometers, the apparatus being provided with an alarm or disabling mechanism which in the event of a deviation from the desired pattern is energized by the position detectors.

The invention is explained hereinafter with reference to the accompanying drawings, in which

Figure 1 is an isodose diagram for a symmetrical radiation distribution,

Figure 2 is a similar diagram for an asymmetrical radiation distribution,

Figure 3 is an isodose diagram for the resultant radiation dose in the case of irradiation with asymmetrical radiation fields from two different directions,

Figure 4 is a schematic perspective view of an irradiation apparatus in accordance with the invention,

Figure 5 shows a cross-section of the apparatus of Figure 4,

Figure 6 schematically represents a synchronous drive of the aperture elements and the patient table in accordance with the invention, and

Figure 7 shows a simple embodiment of a control device for the synchronous drive.

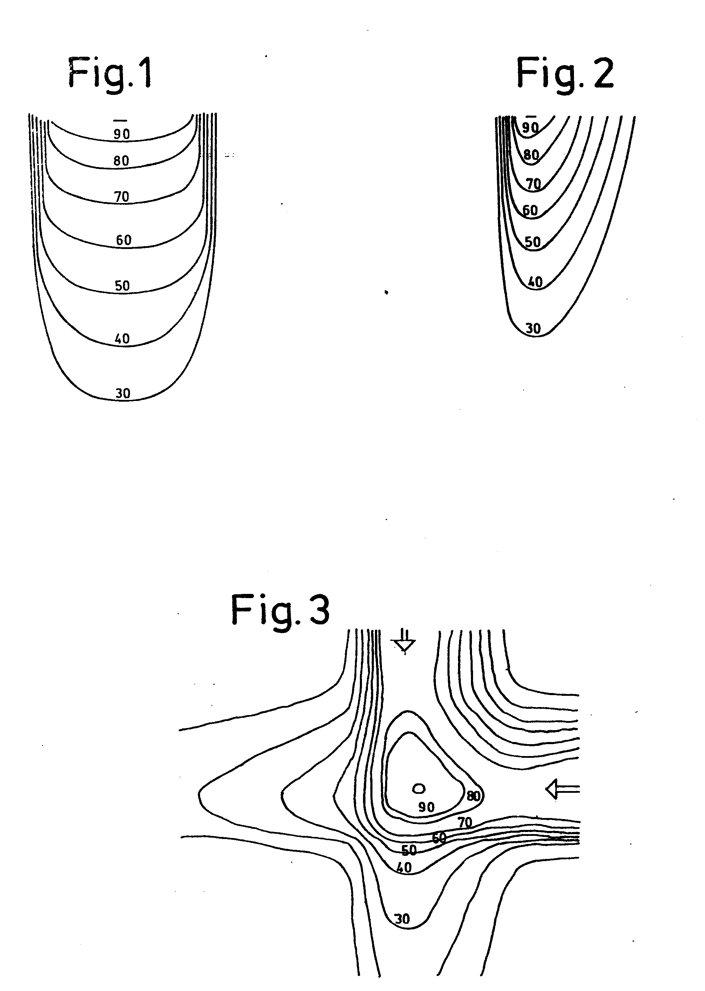

Figure 1 shows an isodose diagram to illustrate the radiation absorption in a homogeneous material, for example a liquid, in the case of irradiation by means of a symmetrical radiation field. Along each curve the radiation absorption has a specific value which is given in per cent of a maximum value (100%).

Figure 2 shows the corresponding isodose diagram in the case of an asymmetrical radiation field which is for
example obtained in a dynamic manner in accordance with the
invention.

Figure 3 shows an isodose diagram which relates
to the resultant radiation absorption in the case of irradiation from two directions, which are perpendicular to
each other, with an asymmetrical field for each of said
directions. A specific isodose curve, for example, 90%,
in the resultant isodose diagram then corresponds to the
shape of for example a tumour to be destroyed to the
greatest possible extent.

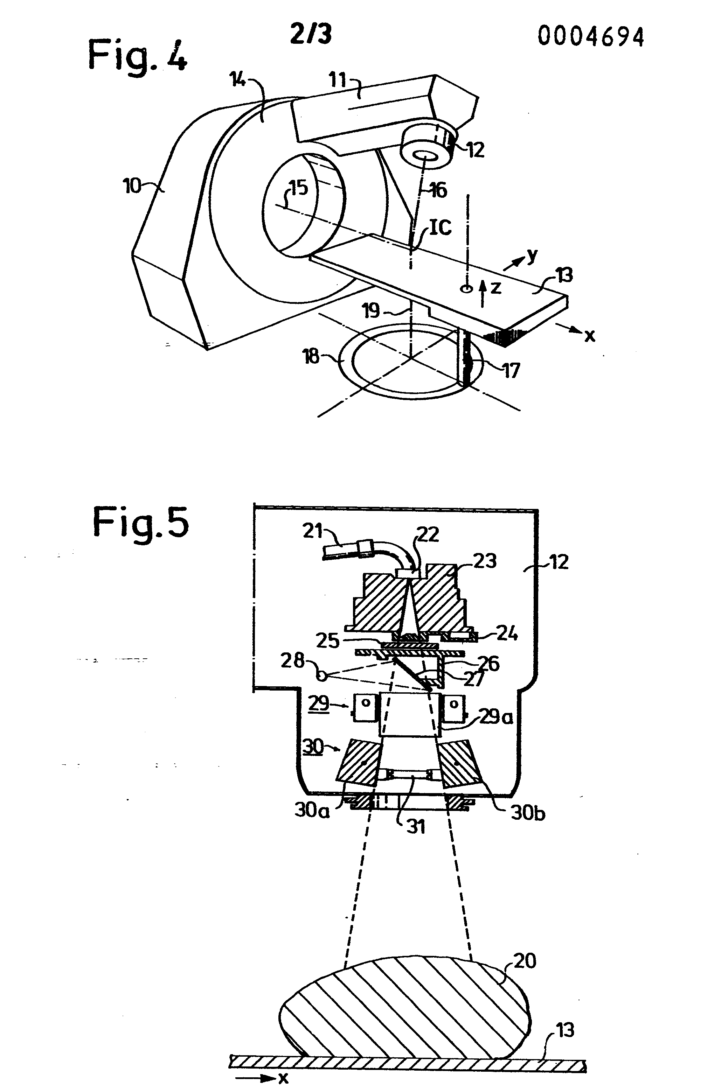

Figure 4 is a perspective view of an irradiation apparatus to which the inventive principles may be
applied. The apparatus comprises a stand 10 with an arm 11
for an irradiation head 12 and a patient table 13 for a
patient to be treated. The arm 11 is secured to a ring 14
which is mounted for rotation in the stand 10. The ring
14 can be rotated about an axis 15 which extends through
the so-called isocentre (IC). When the ring 14 is rotated
and thus when the arm 11 pivots around the patient table
a centre line 16 of a radiation beam emitted by a radiation
source mounted in the head 12 is continuously directed at
the isocentre IC. The head 12 is furthermore rotatable about
the centre line 16. The table 13 is supported by a column
17 which is eccentrically mounted on a rotatable pedestal
18 in the floow in such a way that an axis 19 through the
table extends through the isocentre IC. The patient table
is also rotatable about an axiswhich extends through the
column 17. Finally, the table 13 is movable in a longitudi-
nal direction $\underline{x}$, in a transverse direction $\underline{y}$ and in a ver-
tical direction $\underline{z}$.

Figure 5 is a schematic cross-section of the
irradiation head 12 with the table 13 on which a patient 20
is positioned. In accordance with the invention Figure 5
shows that the heat is positioned so relative to the centre
line 16 of the radiation source that it occupies a position
in which a direction of movement of a pair of aperture
elements 13$\underline{a}$ and 13$\underline{b}$ in the head coincides with a direction

0004694

of movement $x$ for the table. Fig. 5 shows a drift tube 21 for a radiation-producing stream of particles, for example electrons, and an X-ray target 22 with an electron window. The drift tube constitutes the output of a linear electron accelerator and together with the target 22 it forms a radiation source for X-rays or electron radiation. The Figure further shows a fixed primary collimator 23, a filter unit 24, an ionization chamber unit 25, a holder 26 for inter alia a mirror 27, a light source 28 which marks a radiation field, and two aperture limiters 20 and 30, each comprising two aperture elements 29a, 29b and 30a, 30b respectively. The aperture elements are movable away from and towards each other in a substantially linear manner and are coupled to each other in for example known manner, as is shown for the elements 30a and 30b, by means of a coupling rod 31, so that the beam limiting surfaces of the two elements of the pair are always situated at the same distance from the centre line 16 of the radiation beam (symmetrical mode). The same applies to the second pair 29. The adjustment of the two pairs of aperture elements determines the size of the irradiation field to which the patient 20 is exposed.

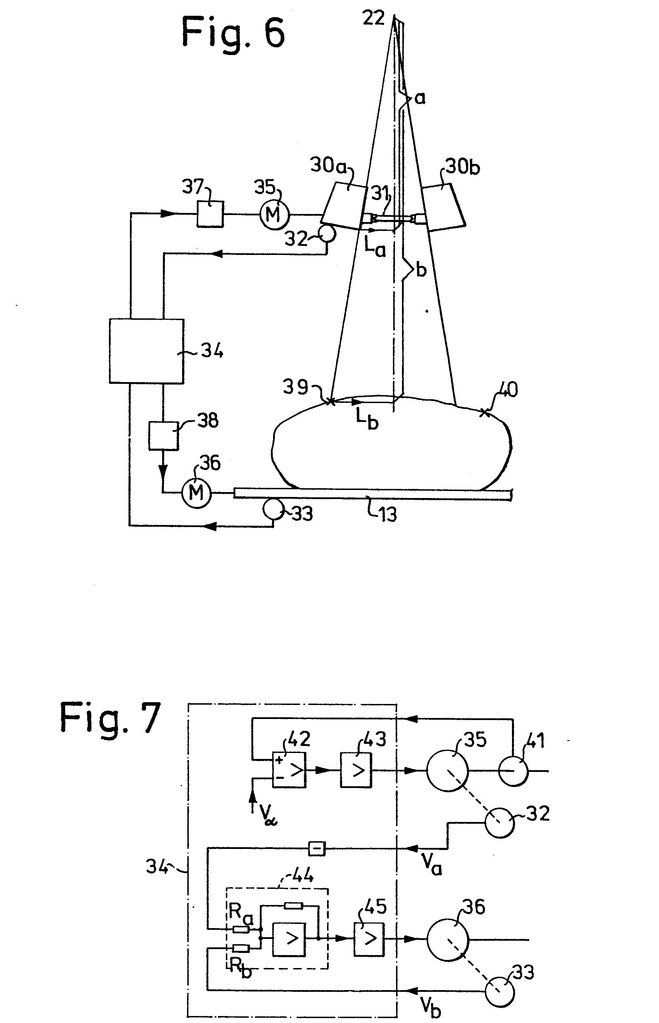

In accordance with the invention the aperture elements are moved pair-wise in synchronism with the table in parallel paths for at least a part of an irradiation period. This is schematically shown in Figure 6, which shows the same cross-section as Figure 5, but now solely with the aperture elements 30a and 30b together with the drive and sensing means for said elements and the corresponding drive and sensing means for the table, as well as a common control device for the table and the aperture elements of the pair 30. In accordance with Figure 6 the instantaneous position of the elements 30a and 30b is detected by a position detector 32, for example in the form of a potentiometer, whilst the instantaneous position of the table 13 is detected by means of a position detector 33. The output signals of the detectors 32 and 33 are applied to a common control device 34. A motor 35 drives the elements 30a and 30b, whilst the table 13 is driven by a motor 36. The control signals for the motors 35 and 36 are applied to the relevant motor by the common control device 34 _via_ 35 and 36 are applied to the relevant motor by the common control device 34 _via_ a modulator/demodulator 37 and 38 respectively. The motors are then controlled in such a way that for example an edge 39 of a radiation field which is defined by the aperture element 30_a_ has a fixed location relative to the patient during the movements of the aperture elements and the table. This may readily be realized by using the known and generally fixed distance between the aperture elements and the radiation source, and the distance between the area to be irradiated and the radiation source. This last-mentioned distance can readily be measured by reading the height setting of the table from a scale graduation, said reading being corrected for the thickness of the patient at the location of irradiation. The table 13 is then controlled so that the distance covered, reckoned from an initial position, always bears the same ratio to the travel of the aperture element 30_a_ as the distances from the irradiated area and the aperture element to the radiation source. If the aperture elements and the table move during the entire irradiation period the radiation dose decreases substantially. If a smaller decrease of the radiation dose is desired, the aperture elements and the table may be moved with a higher speed and may be stopped for a subsequent part of the irradiation period. During the last period the aperture elements have a maximum aperture. At the beginning of the irradiation period the aperture elements are therefore preferably moved from an inner extreme position (maximum aperture) to an outer extreme position (maximum aperture) and when the elements have reached the last-mentioned position they are kept in this position until the end of the irradiation period. The maximum dose is always obtained at the stationary edge 39 of the irradiation field.

Figure 7 shows a simple embodiment of a control device 34. It is now assumed that one of the motors, in the present example the motor 35 which drives the aperture elements of the pair 30, is provided with a tachogenerator 41 which supplies a signal which is proportional to the speed of the motor. The arrangement furthermore comprises a start and stop device (not shown), which initially starts the motors and which stops the motors when a selected specific final position is reached.

In the arrangement of Figure 7 the output signal of the tachogenerator 41 is applied to the positive input of a differential amplifier 42, whose negative input receives a control voltage $V_\alpha$ . The output signal of the differential amplifier 42 is applied to the motor 35 _via_ a control amplifier 43. In the feedback circuit thus formed the motor speed is adjusted through negative feedback until the voltage from the tachogenerator equals the applied voltage $V_\alpha$ , so that the motor 35 has a constant speed which is determined by $V_\alpha$ .

The signal from the position potentiometer 32 associated with the motor 35 is applied, after inversion, to an adder circuit 44 in which said signal passes through an input resistance $R_a$. The signal from the position potentiometer 33 associated with the second motor 36 is also applied to the adder circuit 44, where this signal is passed through an input resistance $R_b$. _Via_ a control amplifier 45 the output signal of the adder circuit 44 is applied to the motor 36. In the closed control circuit thus formed the position of the motor 36 is adjusted depending on the position of the motor 35, until the output signal of the adder circuit 44 is zero. It is assumed that the two potentiometers have the same characteristics and that for generating a signal $V_a$ and $V_b$, which are proportional to the distances $L_a$ and $L_b$ of the aperture elements 30 and the table 13 respectively relative to a reference position (both movements being assumed to be linear). The input resistances $R_a$ and $R_b$ are proportional to the distance _a_ along the centre line between the radiation source and the aperture elements 30 and the corresponding distance _b_ between the radiation source and the irradiated area of the patient (Figure 6). The following equations are then ob-

tained:

$$\frac{V_b}{R_b} - \frac{V_a}{R_a} = 0$$

$$\frac{R_b}{R_a} = \frac{b}{a}$$

$$V_b = k\,L_b; \quad V_a = k\,L_a$$

where $k$ is the proportionality constant of the potentiometers 32 and 33. This yields the following relationship:

$$\frac{L_b}{L_a} = \frac{b}{a} \quad,$$

i.e. the distances $L_a$ and $L_b$ are proportional to the distances $a$ and $b$, at least if the movement of the table and
the aperture element is linear, which was desired.

When the invention is applied to an irradiation
apparatus of the type described irradiation is effected as
follows:

First of all the patient table is adjusted to a
zero or reference position, i.e. a position having the coordinates $x$, $y$ and $z = 0$, and the patient is placed on the
table in an accurately predetermined position. This is
necessary in order to obtain a desired effective radiation
field during all subsequent irradiation cycles from different directions. It is not possible to compensate for an
incorrect positioning on the table by a corresponding movement of the table, because this will result in an incorrect
setting during the subsequent irradiation cycles. In order
to ensure that the patient is in a position on the table
which exactly corresponds to the position on the table
during the localization of the tumour to be irradiated,
when the radiation dose distribution is also determined,
the patient may first be placed in an approximately correct
position by observing an X-ray image of the patient on a
monitor and by comparing this with a radiograph of the
patient in the correct position. Finally positioning is
then effected by means of a number of marks, one of which
is designated by the reference numeral 40 in Fig. 6, which
during diagnosis are made on the patient's body and which for example coincide with light spots from fixed light sources, for example laser sources. Subsequently the irradiation head is rotated until the direction of movement of a pair of aperture elements exactly coincides with the direction of movement of the table, for example in the x-direction. The patient and the radiation source then occupy the correct position relative to each other and irradiation may start. The principal parameter for the irradiation is the total irradiation time and the selected time is set first. Subsequently, the asymmetry of the selected dose distribution is adjusted by selecting the speed of the table and the aperture elements. This setting may for example be expressed as an angle of the equivalent absorption wedge. After this adjustment the apparatus is switched on and irradiation is effected automatically with the selected data. The arm with the irradiation head is then swung to a new position, and the same procedure is repeated etc.

Instead of moving the aperture elements and the patient table with a constant speed, said speed may also be varied in accordance with an absorption wedge having a variable angle. An arbitrary pair of for example two pairs of aperture elements may be moved during the irradiation, provided that the table is moved in a parallel direction. The two pairs may also be moved simultaneously if the table is also moved in both directions. In principle it is also possible to effect irradiation with movable aperture elements and a movable patient table during the movement of the arm which carries the radiation source.

23-2-1979                    1            PHZ 78 004

CLAIMS:

1.          A mecidal irradiation apparatus, provided with a radiation source, means for directing a radiation beam to be produced by the radiation source, an aperture system with at least two aperture elements which are adjustable relative to each other, and a patient table which is adjustable relative to the radiation source, characterized in that a movement mechanism for the patient table and at least one of the aperture elements is controllable by a control device which couples the two movements.

2.          A mecidal irradiation apparatus as claimed in Claim 1, characterized in that the coupling in the moving mechanism to the aperture element and the patient table results in an equal and equally directed angular speed, viewed from the radiation source.

3.          A medical irradiation apparatus as claimed in Claim 1 or 2, characterized in that the two aperture elements which are adjustable relative to each other always have an equal distance to a centre line of the radiation beam and one of the elements is mounted so as to move along with the movement of the patient table.

4.          A medical irradiation apparatus as claimed in Claim 1, 2 or 3, characterized in that the radiation source with the aperture system is rotatable about the patient table and the movement of an aperture element in this respect is adjustable depending on the angle.

5.          A medical irradiation apparatus as claimed in any of the preceding Claims, characterized in that the irradiation apparatus, in order to obtain a synchronous movement of an aperture element and the patient table, is equipped with detectors for supplying signals which are a measure of the instantaneous position of the patient table and the aperture element respectively relative to a reference position.

# Fig.1

# Fig. 2

# Fig. 3

# Fig. 4

# Fig. 5

## Fig. 6

## Fig. 7

3-III-PHZ 78-004